# EUROPEAN PATENT APPLICATION

(11) **EP 4 245 269 A1**
(43) Date of publication of application: **20.09.2023**
(21) Application number: 22162415.8
(22) Date of filing: 16.03.2022
(51) Int. Cl.: A61F 2/44, A61F 2/30

(54) **INTERVERTEBRAL DISC IMPLANT**

(71) Applicant: Cplasty B.V., 1105 BP Amsterdam (NL)
(72) Inventor: Harries, Martin, Sowerby Bridge, HX6 3DA (GB)
(74) Representative: De Vries & Metman

(57) **Abstract**

Intervertebral disc implant comprising a prosthetic nucleus of a hydrogel. The prosthetic nucleus comprises a porous inner core embedded in the hydrogel, e.g., with sections with open cells and sections with closed cells. The porous inner core can for example be made of a 3D printable hydrogel or bio-ink. The prosthetic nucleus further comprises a jacket enclosing the porous inner core and the embedding hydrogel.

## Description

The invention relates to an intervertebral disc implant for repair or replacement of a degenerated intervertebral disc in the spinal column, in particular in the lumbar or cervical spine, e.g., in order to treat the degenerative spinal disc symptom cascade that leads to chronic low back pain or cervical pain. Cervical disc replacement is also a treatment for symptomatic disc herniation with associated arm and hand symptoms.

An intervertebral disc forms a fibrocartilaginous joint between adjacent vertebrae in the vertebral column, allowing controlled movement of the vertebrae, while holding the vertebrae together but preventing hypermobility of the joint. The disc is also a shock absorber for the spine, dissipating energy and protecting other key anatomical organs.

An intervertebral disc comprises three main areas: a gelly nucleus pulposus (NP) forming the core of the disc; a fibrous annulus fibrosus (AF) surrounding the NP, and the cartilaginous endplates (EP) sandwiching the NP. The NP spaces the vertebrae, absorbs impact loads and hydraulically distributes pressure, in conjunction with the AF, across the intervertebral disc space so as to prevent stress concentrations causing damage to the adjacent vertebrae and other vital organs. The AF has a stiff, layered structure for withstanding tensile, shear and torsional forces and works sym-biotically with the NP to disseminate axial load into radial tension.

Several factors contribute to degeneration of an intervertebral disc, including aging, genetic inheritance, inadequate metabolite transport, and loading history, eventually leading to a loss of tissue hydration and functionality that can entail structural failure of the AF and subsequent herniation of the NP.

US 5,047,055 discloses implants comprising hydrogel cores with a shape generally conforming to the shape of the NP. WO 00/59412 discloses similar hydrogel cores, which are surrounded by a constraining jacket.

A drawback of these prior art implants is that they expand uniformly, so the whole intervertebral space height between the two adjacent vertebrae will be increased by the same distance. This flattens the spinal curvature, creating a kyphos. The natural inward curvature of the lumbar and cervical regions of the human spine - known as lordotic or secondary curvatures - transfers the person's weight load over the pelvis, allowing for a more efficient walking gait. Normal lordotic curvatures result from a difference in the thickness between the front and back parts of the intervertebral disc. Flattening these lordotic curvatures can cause constant and painful tension in the spinal muscles. Restoring the natural lordotic curvature restores the sagittal profile which maintains the head in a central location above the hips and knees. An unbalanced sagittal profile subjects both these joints to increased stresses.

It is an object of the invention to provide an intervertebral disc implant for replacing at least the NP of an intervertebral disc providing improved compressive strength and biomimicking, e.g., enabling it to contribute to lordotic shaping.

The object of the invention is achieved with an intervertebral disc implant comprising a prosthetic nucleus of a hydrogel. The prosthetic nucleus comprises a porous inner core embedded in the hydrogel. While the hydrogel hydrostatically distributes loads, the porous inner core can be shaped to optimize mutual orientation of the two adjacent vertebrae to restore or improve the lordotic curvature.

In a particular embodiment, the porous inner core has a structure with open cells. The open cells allow absorption of the surrounding hydrogel providing improved structural integrity of the intervertebral disc implant.

Te porous inner core may also comprise closed cells, e.g. have one or more closed cell sections with a closed cell density which is higher than the closed cell density of one or more other section, e.g., open cell sections. For instance, the closed cell sections may have a cell structure with more than 50 % of its cells being closed.

The closed cells protect against fragment retro-pulsion or sequestration, i.e., displacement of any hydrogel fracture fragment into the spinal canal, thereby potentially causing spinal cord injury. The closed cells block passage of such fracture fragments through the intervertebral disc implant. To this end, the closed cells can for example form a closed wall at a posterior side of the intervertebral disc implant.

The viscoelastic dynamics in the closed cell section will be different from those in the open cell section of the porous inner core. The closed cell section will therefore be stiffer than the open cell section. Positioning the stiffer closed cell section at the posterior and anterior side facilitates good angular movement, while minimizing the dynamics in the facet joints. Under compression the closed cell section of the porous inner core maintains height of the intervertebral disc space.

The porous inner core can be used to support restoration of the natural lordotic curvature. To this end, the porous inner core can be provided with a wedge shape. Whereas the intervertebral disc implant as a whole may have a shape conforming to the natural NP, i.e., substantially domed in top view, the porous inner core and the intervertebral disc implant as a whole may have a wedge shaped sagittal cross section, e.g., with rounded edges. The wedge shaped sagittal cross section may have a larger height at the anterior side of the intervertebral disc implant and a lower height at the posterior side. The wedge angle (i.e., the dihedral angle between the upper face and the lower face of the porous inner core) may for example be at least 3 degrees, e.g., at least 5 degrees, e.g., at most 9 degrees, e.g., about 6 degrees.

The porous inner core can for example be customized and made on demand by computer aided manufacturing, in particular by rapid prototyping. Suitable rapid prototyping processes include stereolithography, fused deposition modelling, laminated object modelling, selective laser sintering, computer aided milling and, in particular, 3D printing. Combinations can also be used. For instance, a slightly oversized product may be printed and subsequently milled to improve dimensional accuracy or to obtain a desired surface finishing.

In this respect, it is noted that US 2021/093457 discloses 3D printed intervertebral disc implants designed for matching and maintaining a desired spinal lordosis.

The porous inner core can for example be made of a hydrogel, e.g., a hydrogel that is different from the hydrogel embedding the porous inner core. The hydrogel can for example be a printable hydrogel, while the embedding hydrogel is a flowable hydrogel. An overview of 3D printable hydrogels and suitable 3D printing techniques is presented in the article *"*Review of 3D printable hydrogels and contracts", Li c.s., Materials & Design, vol. 159, p. 20-38, 2018. Suitable hydrogels for 3D printing include biocompatible materials that integrate well into the body, such as alginate and collagen, e.g. collagen-II-HA. Acombination of polylactic acid (PLA) with gum-polyethylene glycol diacrylate (GG-PEGDA) can also be used. While PLA provides biocompatibility and water retention, GG-PEGDA enables printability and supplies enhanced strength. Polyethylene glycol (PEG) combined with sodium alginate forms a suitable 3D printing ink producing highly stretchable hydrogels tougher than natural cartilage and showing high cell viability. A 3D printing ink particularly suitable for printing porous structures is silk fibroin, optionally modified with glycidyl methacrylate and combined with elastin. Other suitable 3D printing inks comprise chitosan and/or hyaluric acid with (nano) cellulose fibres. Bio-inks - i.e. 3D printing inks comprising cells in a matrix materials, such as a biopolymer gel or a hydrogel - can also be used.

Prior to 3D printing, a scan, such as a CT and/or MRI scan can be made of the intervertebral disk space, where the implant is to be placed. Based on this information, a digital model of the implant can be generated, e.g., having a shape optimized for restoring the desired lordotic curvature. The stiffness of the open cell and closed cell sections of the porous inner core can be customized, e.g., in function of the patient's size and weight.

Alternatively, the porous inner core can be made of a woven textile, e.g., of PET, a polyurethane foam or any other suitable foam material or a medical grade elastomer, such as polyurethane, silicone or EPDM.

The porous inner core is embedded in a hydrogel, in particular a flowable hydrogel. A hydrogel is a crosslinked polymeric material with hydrophilic polymeric chains capable of holding large amounts of water in their three-dimensional networks. The hydrogel embedding the porous inner core is partly absorbed into the porous inner core and can for example be a hydrogel based on polyvinyl alcohol (PVA), 2-hydroxyethyl methacrylate (HEMA), poly hydroxyethyl methacrylate (PHEMA), or polyethylene glycol (PEG).

In a specific embodiment, the hydrogel is a hydrogel expanding from a dehydrated shape to a hydrated shape by in-situ absorption of moisture after positioning the intervertebral disc implant in the intervertebral disc space. This facilitates insertion of the dehydrated intervertebral disc implant into the intervertebral space via an opening in the annulus. This makes the intervertebral disc implant particularly suitable for replacing an NP in the lumbar region, which requires posterior implantation, where access to the intervertebral space is limited by the nerve root. After rehydration the prosthetic nucleus provides intradiscal height and forms an adaptive centre of rotation. The hydrogel outer layer hydraulically redistributes loads.

The viscoelastic dynamics of the outer core controls the local centre of rotation. The centre of rotation at each spinal level varies from the next, as does the arc of motion. Under extension (bending backwards) and flexion (bending forwards), the centre of rotation moves slightly in sagittal direction. Under lateral bending, the centre of rotation moves slightly in a lateral direction. Under axial rotation, the centre of rotation resides outside the porous inner core closer to the posterior side of the spinal cord. These movements mimic behaviour of the natural intervertebral disc. Adapting the local centre of rotation can diminish excessive load transfer through the motion segment.

Optionally, the hydrogel can be seeded with mesenchymal cells promoting regeneration into a natural NP.

The prosthetic nucleus comprises an outer jacket fully enclosing the hydrogel and the porous inner core. The jacket can for example be made of tightly woven high molecular weight, high tenacity polymeric fabric, such as a polyethylene, e.g., UHMWPE, or polyester, such as polyethylene terephthalaat (PET). Alternatively, polyamide or any other suitable high tenacity polymeric material can be used, just as ceramic fibres.

The jacket can be made of fibres having little tensile elasticity. As a result, the jacket defines a generally fixed maximum volume, which may be less than an unconstrained volume of the prosthetic nucleus, when the hydrogel is completely hydrated without constraint. Thus, because the prosthetic nucleus has a natural, fully hydrated volume greater than the filled jacket, the jacket will be tight about the prosthetic nucleus when the hydrogel is rehydrated. The volume difference between the expanded jacket and the rehydrated prosthetic nucleus prevents the hydrogel from reaching its natural hydration level. Consequently, the hydrogel of the prosthetic nucleus will have a constant affinity for absorbing ambient moisture.

The jacket may be an open weave or closed weave textile. In this respect, "open weave" is defined as a weave allowing ingrowth of fibroblasts within its structure, which can be used to mimic the endplate. Closed weave only allows growth with at most a technically negligible degree of in-growth and can in particular be used posteriorly to mimic the posterior longitudinal ligament.

The jacket can be shaped to allow more expansion in the central third of the intervertebral disc implant, for example for further adjusting the local center of rotation.

To promote ingrowth of tissue, the jacket material can be provided with a bio-coating or bio-adhesive. A particular suitable material for bio-coating of the jacket comprises mesenchymal stem cells, e.g., a bio-coating on basis of polycaprolactone and/or silk to be mixed into yarn of the textile so as to form a hybrid textile with enhanced regenerative properties, especially useful at the interface of the endplate and the vertebral body.

To enable accurate positioning of the intervertebral disc implant in the targeted intervertebral disc space, the jacket can be provided with radiographic markers, for example one or more lines in sagittal direction and/or one or more lines in a direction perpendicular to the sagittal direction. Such markers can for example be woven into the jacket. Suitable fibres that can be woven into the jacket as a marker, are for example black fibres of medical grade ultrahigh molecular weight polyethylene (UHMWPE), such as Dyneema Purity^{®} black fibre, available from DSM.

After positioning the intervertebral disc implant, the height of the intervertebral disc will be restored. This will put the residual natural AF under constant mechanical stress, triggering the AF to heal and regenerate. However, if the natural AF is too compromised, an AF patch can be used as a scaffold, such as a fibrous scaffold plate. Such a fibrous scaffold plate can for example be placed at a posterior and/or anterior side of the intervertebral disc space. Residual AF will gradually grow into the fibrous scaffold plate until the AF is back into its original natural volume.

The fibrous scaffold plate can be connected to adjacent vertebrae, e.g., by a primary mechanical fixation, such as a textile anchor or a screw, e.g., using caspar pins. To this end, the fibrous scaffold plate can be provided with openings or eyelets for receiving the anchors or fasteners. Fibrin glue can also be used that it provides a good seal and connection between any residual annulus.

Optionally, the fibrous scaffold plate is mainly made of fibres with a main vertical orientation, optionally bond by transversal fibres. To mimic the lamellae structure of the AF, the fibres can converge in upward direction.

The fibrous scaffold plate can for example be made of tightly woven high molecular weight, high tenacity polymeric fabric, such as a polyethylene, e.g., UHMWPE, or polyester, such as polyethylene terephthalate (PET). Alternatively, polyamide or any other suitable high tenacity polymeric material can be used, just as carbon fibres, ceramic fibres, etc., or mixtures thereof.

Ingrowth of tissue can be promoted by providing the fibrous scaffold plate with a bio-coating or bio-adhesive. Particularly suitable are bio-coatings comprising platelet rich plasma.

If the fibrous scaffold plate is applied in the cervical region, it can be used on the anterior two thirds of the intervertebral disc space.

Anterior application in the lumbar region is very complicated due to the aorta and vena cava running anteriorly along the spine. Anterior surgery can be performed between the aortic bifurcation which makes it a complicated approach with limited possibilities. Therefore, a posterior approach is more appropriate in this region, e.g., placing the fibrous scaffold plate on a posterolateral corner of the disc.

Another option is a lateral approach. In such a case, the fibrous scaffold plate may have a trapezoid shape mimicking local lordotic curvature of the lumbar spine by copying the wedge shape of the intervertebral disc space in side view.

The fibrous scaffold plate may be applied along the full circumference of the intervertebral disc space, e.g., at least two third of the circumference, e.g., at least half of the circumference, e.g., at least 10 % of the circumference.

Optionally, the hydrogel porous inner core has a cannula which projects out from the porous inner core. The cannula can be used to handle the porous inner core during insertion. It can also be used to inject the hydrogel for forming the outer layer, until the jacket is completely filled. The cannula is then removed.

In an alternative embodiment, the targeted intervertebral disc first needs to be decompressed, by pushing apart the two adjacent vertebrae, e.g., using a detractor with caspar pins and removing the natural disc material compressing the nerve root. The dehydrated jacketed intervertebral disc implant is then inserted as a whole. The hydrogel outer layer will then re-hydrate in situ and restore height of the intervertebral disc space.

The intervertebral disc implant can be used in any stage of degeneration of the intervertebral disc. Currently most intervertebral disk replacements are in the upper age group, when the degeneration is such that it causes physical symptoms. The present intervertebral disc implant does not require complete removal of the NP or AF and can be used for augmenting the anterior motion segment, rather than completely removing and replacing the degenerated natural intervertebral disc. It can be used before the degeneration becomes too severe, i.e., providing a prevention or slowing of an ongoing degeneration.

Instead of completely replacing the natural intervertebral disc in a late stage of degeneration, the design of the intervertebral disc implant can be customized and downsized, so the existing intervertebral disc can be augmented by repairing the AF, and/or by partial repair of the NP rather than completely replace.

Degeneration starts in the anterior motion segment side but in a very late stage degeneration will eventually affect the posterior motion segment side, more particularly the facet joints. If the facet joints have been affected the degeneration must be treated by spinal fusion. Spinal fusion is a surgical treatment in which two or more vertebrae are fused. This prevents any movement between the fused vertebrae. A fusion cage is placed between the two vertebrae allowing ingrowth of bone tissue to fuse the two vertebrae. To this end, a porous body can be used corresponding to the porous inner core of the intervertebral disc implant of the present disclosure, which porous body can be made of a non-compressible bio-compatible material, such as PEEK, titanium, bioceramics, or carbon materials, such as graphene or graphite, in particular aerographene or aerographite. The porous body is used without an embedding hydrogel and without a jacket. Optionally, a fibrous scaffold plate can be used to restore the AF, as described above. The open cells of the porous body allow in-growth of bone tissue. The closed cells of the porous body protect against osteophyte formation encroachment of the foramen.

Patients with a high grade degeneration at one level, usually also show a lesser degree of degeneration at adjacent levels. In such cases, the level of the late stage of degeneration can be treated by spinal fusion with an implant having a porous inner core of a non-compressible bio-compatible material, whereas the adjacent levels can be treated by disc replacement using an implant having a hydrogel porous inner core.

So the invention provides a system to treat different degrees of intervertebral disc degeneration within the same patient. A surgeon can treat any stage of degeneration within one patient with a single system in accordance with the invention.

The invention is further explained with reference to the drawings, showing exemplary embodiments.
Figure 1: shows an exemplary embodiment of an intervertebral disc implant in transversal cross section;
Figure 2: shows the porous core of the implant of Figure 1;
Figure 3A: shows an exemplary embodiment of a fibrous scaffold plate at a posterolateral side of a lumbar intervertebral disc space;
Figure 3B: shows a fibrous scaffold plate at an anterior side of a lumbar intervertebral disc space;
Figure 3C: shows a fibrous scaffold plate at a lateral side of a lumbar intervertebral disc space;
Figure 3D: shows a fibrous scaffold plate at an anterior a side of a cervical intervertebral disc space.

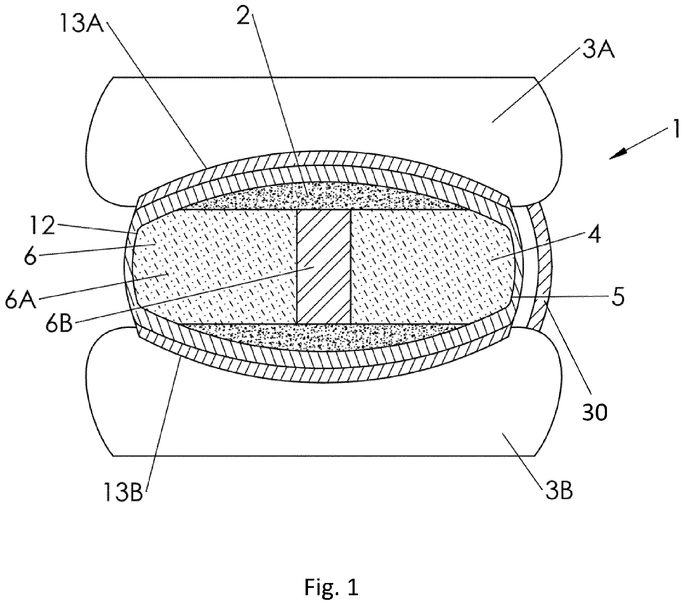

Figure 1 shows a transversal cross section of an intervertebral disc implant 1 in an intervertebral disc space 2 between two vertebrae 3A, 3B. The intervertebral disc implant 1 replaces a nucleus pulposus (NP) of a natural intervertebral disc. The natural NP serves to space the vertebrae 3A, 3B and to absorb and hydraulically distribute pressure and impact loads. To mimic this, the intervertebral disc implant 1 has a prosthetic nucleus 4 of a flowable and biocompatible hydrogel 2 in a jacket 5 of a fibrous PET material, biasing the hydrogel of the prosthetic nucleus 4 into a desired shape, e.g., to support lordotic shaping.

The prosthetic nucleus 4 further comprises a porous inner core 6 fully embedded in the biocompatible hydrogel 2. The biocompatible hydrogel 2 forms an outer layer around the porous inner core 6. The porous inner core 6 has a section 6A with open cells or pores absorbing the biocompatible hydrogel which supports structural integrity of the intervertebral disc implant 1. The porous inner core 6 also has a section 6B with closed cells forming a posterior wall blocking passage of vertebral fracture fragments through the intervertebral disc implant 1 to the spinal cord (not shown). The closed cell section 6B and the open cell sections 6A form an integral part and the transition between the sections may not be a sharp as shown in the drawing. In fact the all sections will comprise open cells and closed cells but the closed cell section 6B will typically have a higher density of closed cells than the open cell sections 6A.

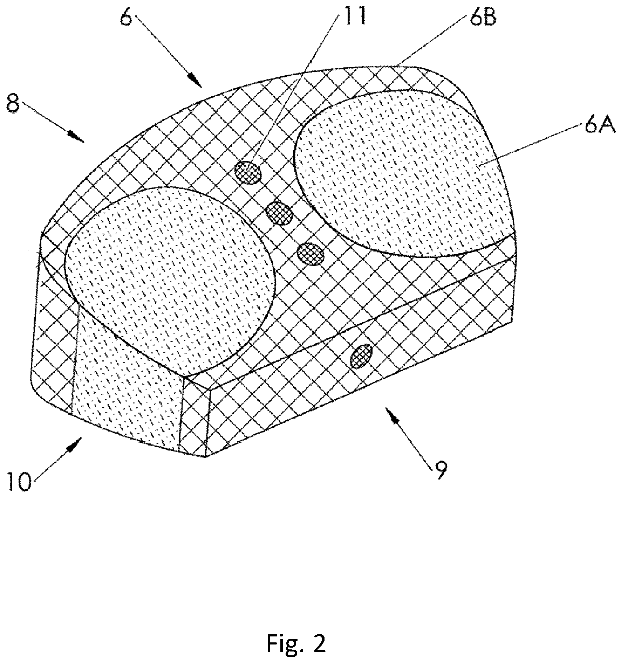

In Figure 1, the porous inner core 6 is shown with a substantially rectangular cross sectional shape. In sagittal cross section (perpendicular to the cross section shown in Figure 1), the porous inner core 6 can for example be substantially wedge-shaped, so as to force the adjacent vertebrae 3A, 3B to a mutual orientation restoring the natural lordotic curvature. The porous inner core 6 is shown separately in perspective view in Figure 2, and has relatively high side face 8 at the anterior side, a lower side face 9 at the posterior side and wedge shaped lateral side faces 10.

The foam-like structure of the porous inner core 6 with open and closed cells can for example be made by 3D-printing of a printable hydrogel or bio-ink. This also allows customized shaping of the porous inner core to optimize lordotic re-shaping.

In the shown exemplary embodiment, the closed cells 6B of porous inner core form a T-shaped or anchor shaped section with an anterior flange and a posterior flange bridged by a centrale web section. The open cells 6A form two oval bodies fitting between the web plate and the anterior and posterior flanges. Other distributions of open and closed cells can also be used, in particular to optimize and tailor the visco-elastic dynamics of the inner core 6 for a specific case.

The embedding biocompatible hydrogel 2 is absorbed by the open cells 6A of the porous inner core 6. To facilitate an more even distribution of the biocompatible hydrogel through the porous inner core 6, the closed cell sections 6B can be provided with channels or fenestrations 11.

The intervertebral disc implant 1 is surrounded by the annulus fibrosus (AF) 12 and sandwiched between the cartilage endplates 13A, 13B of the adjacent vertebrae 3A, 3B.

The intervertebral disc implant 1 can for example be implanted by means of a cannula (not shown). In a first step, the porous inner core 6 is inserted between the two vertebrae 3A, 3B. The porous inner core 6 is within the jacket 5 but without the hydrogel forming the outer layer 7 of the prosthetic nucleus. The porous inner core 6 comprises a cannula which is used for handling the porous inner core during insertion. In a next step, a hydrogel is injected via the cannula. The hydrogel 2 is partly absorbed by the open cells 6A of the porous inner core 6 and partly forms an outer layer around the porous inner core 6. Injection of hydrogel is stopped after sufficient hydrogel is injected to provide the desired height of the intervertebral disc space 2. In a final step, the cannula is removed.

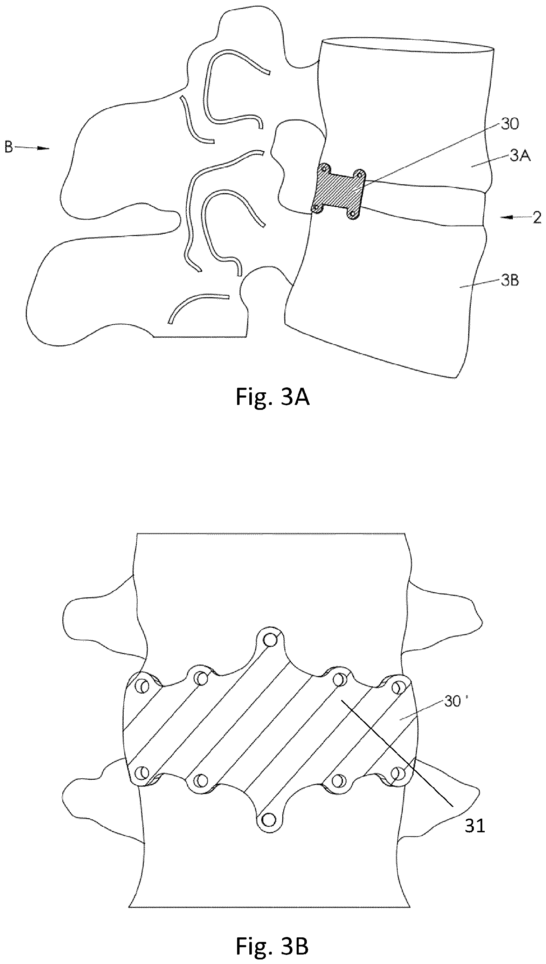

In Figure 1, the AF 12 is still intact. If the AF 12 also needs to be restored, a fibrous scaffold plate can be used stimulating regrowth of the AF tissue. Figures 3A-D show examples of such fibrous scaffold plates 30 in different positions.

In the lumbar region (Figures 3A-C), the AF 8 spans the full circumference of the intervertebral disc space 2 in top view. To replace an intervertebral disc in the lumbar region, the surgeon typically operates from the posterior side B. Parts of the facet joints may need to be removed to stay at safe distance from the spinal cord and reach the intervertebral disc space at a lateral side. The fibrous scaffold plate 30 can then be attached to a posterolateral corner of the two respective vertebrae.

Figure 3B shows a fibrous scaffold plate 30' applied at the anterior side of an intervertebral disc space 2 in the lumbar region. Such an anterior approach is exceptional since the surgeon must approach the intervertebral disc space 2 through the patient's belly, which usually has substantial impact on the patient's recovery. Here, a larger scaffold plate can be used, since the surgeon has better access.

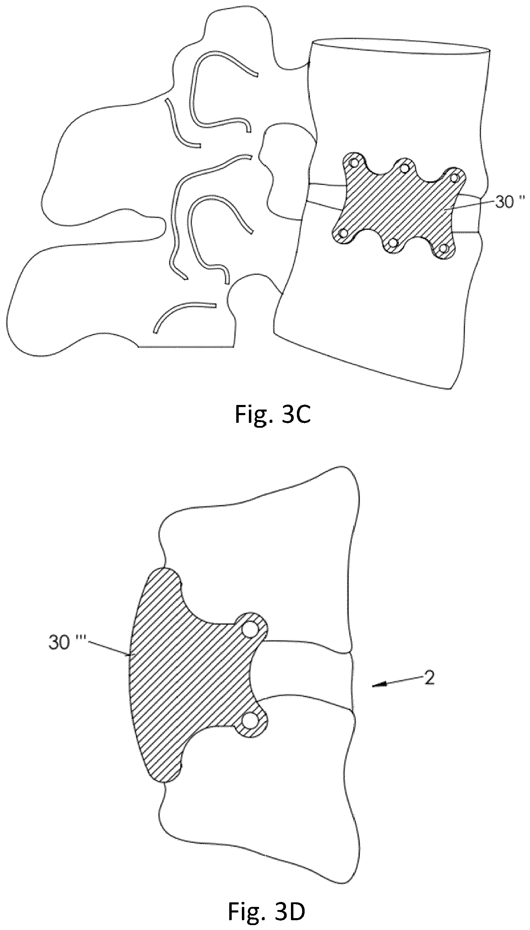

Figure 3C shows an alternative lateral approach. In this case, the surgeon approaches the intervertebral disc space 2 from the side. Due to the wedge-shape of the intervertebral disc space 2, the fibrous scaffold plate 30" is rhomboid shaped as it needs to be higher at the anterior side than at the posterior side.

Figure 3D shows a fibrous scaffold pate 30‴applied in the cervical region. In this region, the intervertebral disc space 2 can be reached form the anterior side without disturbance of internal organs. Moreover, in the cervical region, the AF only spans the anterior two third of the circumference of the intervertebral disc space 2, so anterior application is the most appropriate approach.

The fibrous scaffold plates 30', 30", 30‴are dimensioned in accordance with the actual anterior, posterior or lateral position. The fibrous scaffold plates are woven or non-woven plates of bio-compatible fiber material, in particular PET. In the cervical region (Figure 3D), the fibers converge in an upward direction to mimic the lamellae structure of the anterior cervical AF tissue.

The fibrous scaffold plates are provided with eyelets 31 or similar openings for receiving fasteners to anchor the fibrous scaffold plates to the two adjacent vertebrae.

## Claims

1. Intervertebral disc implant comprising a prosthetic nucleus of a hydrogel,
wherein the prosthetic nucleus comprises a porous inner core embedded in the hydrogel.

2. Intervertebral disc implant according to claim 1, wherein the porous inner core has a structure with open cells.

3. Intervertebral disc implant according to claim 2, the structure of the porous inner core further comprising closed cells.

4. Intervertebral disc implant according to claim 2, wherein the closed cells form a posterior wall.

5. Intervertebral disc implant according to any one of the preceding claims, wherein the porous inner core is made of a hydrogel or bio-ink.

6. Intervertebral disc implant according to claim 5, wherein the porous inner core is made by a rapid prototyping processes, e.g., a rapid prototyping process selected from stereolithography, fused deposition modelling, laminated object modelling, selective laser sintering, and/or 3D printing.

7. Intervertebral disc implant according to any one of the preceding claims, wherein the prosthetic nucleus further comprises a jacket enclosing the hydrogel and the porous inner core.

8. Intervertebral disc implant according to claim 7, wherein the jacket is made of a fibrous material, e.g., of polyethylene terephthalate (PET) fibers.

9. Intervertebral disc implant according to claim 7 or 8, wherein the jacket is at least partly coated with a bio-coating, for example a bio-coating comprising mesenchymal stem cells.

10. Intervertebral disc implant , optionally according to any one of the preceding claims, comprising a fibrous scaffold plate connected or connectable to two vertebrae defining an intervertebral disc space, the fibrous scaffold plate extending along at least a part of the contour of the intervertebral disc space.

11. Intervertebral disc implant according to claim 10, wherein the fibrous scaffold plate is connected to the two adjacent vertebrae, e.g., by a mechanical connection, such as an anchor, and/or by means of an adhesive.

12. Intervertebral disc implant according to claim 10 or 11, wherein the fibrous scaffold plate comprises a bio-coating, e.g., comprising platelet rich plasma.

13. System to treat different degrees of intervertebral disc degeneration at different spinal levels within the same patient, comprising one or more of the intervertebral disc implants according to any one of the preceding claims and a fusion cage comprising a porous body of a non-compressible bio-compatible material, such as PEEK, titanium, or carbon materials, such as graphene or graphite, in particular aerographene or aerographite.

14. Fusion cage for spinal fusion, comprising a porous body non-compressible of a bio-compatible material, such as PEEK, titanium, or carbon materials, such as graphene or graphite, in particular aerographene or aerographite.
